# EUROPEAN PATENT SPECIFICATION

(11) **EP 3 295 855 B1**
(45) Date of publication and mention of the grant of the patent: **26.06.2019**
(21) Application number: 16382434.5
(22) Date of filing: 16.09.2016
(51) Int. Cl.: A47L 15/00, A47L 15/42, D06F 35/00, A47J 31/60, A61L 2/22

(54) **HOUSEHOLD APPLIANCE WITH AN AEROSOL GENERATOR AND METHOD FOR OPERATING THE SAME**
HAUSHALTSGERÄT MIT EINEM AEROSOLGENERATOR UND VERFAHREN ZUM BETRIEB DAVON
APPAREIL MÉNAGER AVEC GÉNÉRATEUR D'AÉROSOL ET SON PROCÉDÉ DE FONCTIONNEMENT

(43) Date of publication of application: 21.03.2018
(73) Proprietor: BSH Hausgeräte GmbH, 81739 München (DE); BSH Electrodomésticos España, S.A., 50016 Zaragoza (ES)
(72) Inventor: Bischof, Andreas, 10407 Berlin (DE); Lazaro Villarroya, Guillermo Alberto, 50005 Zaragoza (ES); Mischo, Horst, 54295 Trier (DE); Paintner, Kai, 86465 Welden (DE); Sanz Naval, Javier, 50193 Zaragoza (ES); Schepers, Klaus, 35619 Braunfels (DE)

(56) References cited:
- EP-A1- 3 216 378
- WO-A1-2014/154432
- DE-A1-102011 083 572

## Description

The invention relates to a household appliance with a means to generate an airstream and an aerosol generator comprising a nebulizer and a liquid reservoir having an interior catalytically active surface. In particular the invention relates to a household appliance with a means to generate an airstream and an aerosol generator comprising a nebulizer and a liquid reservoir having at least one interior catalytically active surface containing a polyoxometalate and a method for operating the same.

Household appliances may come into contact with dust, dirt, food or humidity or may be touched by animals or during use. Problems concerning hygiene may thus arise from microorganisms that can attach to household appliances and proliferate. In particular, contaminated and hygienically objectionable household appliances or at least household appliances having discolorations may result. In the worst case this may be even dangerous to health. Regular cleaning of a household appliance is thus recommended. However, convenience and effectiveness of such a cleaning may be determined by the kind and amount of the contamination. In some cases a biofilm may be formed or has already formed. Biofilms, which consist of organic substances such as microorganisms and nutrients, lead to bad odor and/or visible contamination.

This is in particular the case for household appliances which come into contact with food stored, processed or prepared inside the household appliance. Even vacuum cleaners tend to exhibit contamination with microorganisms.

In general, articles which have been soiled in various ways are cleaned in water-bearing household appliances. Food remains arise in dishwashers and the range of dirt occurring in laundry items to be cleaned in washing machines is typically even greater. Something that all water-bearing household appliances have in common is that in the damp and warm atmosphere, in particular at less accessible sites, dirt can arise and accumulate. This dirt can be a good nutrient medium for organisms such as bacteria or fungi.

A general problem of household appliances is that microorganisms tend to grow faster and easier in a damp atmosphere, in particular at less ventilated sites that are often also less accessible. For example condensed water in a fridge may support the growth of microorganisms. Reservoirs for liquids such as water and milk, e.g. in coffee machines, are prone to the growth of microorganisms.

It would thus be desirable to provide a household appliance that can be operated more hygienically and wherein measures can be taken to avoid the formation of biofilms.

Various measures for removing and/or preventing biofilms are known from the prior art. In particular, sometimes, machine cleaning programs are offered which remove built-up dirt at high temperatures with the assistance of washing agents and sometimes with increased liquor levels and/or at raised drum rotation speeds, i.e. with an increased input of mechanical energy. Also known is the use of ozone to remove organic dirt.

It has also previously been proposed to remove organic dirt with the aid of UV-C radiators using a through-flow principle, the removal taking place in such a manner that microorganisms in such dirt are finally killed by damaging their genetic material. Photocatalytic methods are also known, for example, the use of titanium dioxide coatings for deodorizing, disinfecting and cleaning. In this process, the catalyst needs to be activated by means of UV radiation.

Also known are measures for killing microorganisms using Ag+ or Cu+ ions in the washing liquor and in the surfaces of the materials coming into contact with the washing liquor.

Other methods are directed to thermal killing of any microorganisms present by increasing the temperature at the surfaces of the components coming into contact with the washing liquor, via direct or indirect energy transfer (water, steam, microwaves).

A disadvantage of these known methods and measures is the high energy consumption and the sometimes high apparatus and/or operating costs for achieving truly relevant effects. In the case of the use of Ag+ or Cu+ ions, disadvantageous effects with regard to groundwater and waterway pollution also arise. In some methods, potentially health-endangering agents, for example ozone or UV radiation, are used so that additional safety measures are required.

The use of polyoxometalates in various fields is known, for example in analytical and clinical chemistry, in catalysis (including photocatalysis), in biochemistry (inhibition of electron transfer processes), in medicine (antitumor and antiviral activity) and in the manufacturing of integrated circuits. Polyoxometalates are known as oxidation catalysts most of all in the paper and plastics industry.

The publication EP 3 216 378 A1, which forms prior art according to Art. 54(3) EPC, discloses a household appliance with a liquid reservoir having at least one interior catalytically active surface containing a polyoxometalate, which has the formula A₃PW₄O₂₄ or A_{q+3}PV_{q}M_{o12-q}O₄₀, where q is 1 or 2, A is selected from one or more cations selected from the group consisting of (XR¹R²R³R⁴)⁺, wherein at least one of the groups R¹, R², R³, and R⁴ is a substituted or non-substituted alkyl, or cycloalkyl group with at least five carbon atoms and wherein X is selected from among nitrogen and phosphorus.

The publication EP 1 141 210 B1 discloses a method for bleaching laundry items or household surfaces wherein a washing agent which contains polyoxometalates is brought into contact with the soiled substrate. Air serves as the primary source of oxygen atoms for bleaching.

The publication DE 10 2009 026 712 A1 discloses a household appliance having at least one component, which has a surface that can be affected by organic dirt, said surface having a photocatalyst, a photosource for irradiating the photocatalyst with an activating electromagnetic radiation being associated with said surface, the surface being formed from a primary formed first material in which the photocatalyst is dispersed. Materials with titanium dioxide and modifications thereof are disclosed in great detail as photocatalysts.

The publications EP 2 761 073 B1 and US2014/231363 A1 disclose a water-bearing domestic appliance having a container for receiving objects to be cleaned and at least one inner surface containing a catalytically active substance, said surface being disposed inside the domestic appliance, wherein the catalytically active substance is a polyoxometalate and the inner surface comes into contact with water to be cleaned during operation of the domestic appliance. Preferably the polyoxometalate is tungstate and even more preferably the tungstate is modified by titanium.

The publications DE 10 2013 205 302 A1 and WO 2014/154432 A1 disclose a household appliance which comprises at least one catalytically effective substance in a surface, wherein the catalytically effective substance is a polyoxometalate that is comprised in an inner and/or outer surface of the household appliance, provided that the polyoxometalate is comprised at least in an outer surface of the household appliance if the household appliance is a water-bearing household appliance having a container for receiving objects to be cleaned.

The publication WO 2014/122225 A1 discloses the use of a heteropolyoxometalate of the Formula (I), (II) or (III)

A_{q+3}PV_{q}Z_{12-q}O₄₀ (I),

A₆P₂Z₁₈O₆₂ (II),

or

A₃PZ₄O₂₄ (III)

wherein Z is selected from Mo or W,
index q = 0, 1, 2 or 3, and
A is selected from one or more cations and comprises at least one cation selected from the group consisting of quaternary ammonium cations, quaternary phosphonium cations and tertiary sulfonium cations for providing self-cleaning, stripping, disinfecting, self-sanitizing, biocidal, antimicrobial, and/or deodorizing properties to at least part of a substrate or a surface of a substrate or to a coating or for decomposition and/or degradation of organic materials. In Formula (I), Z is preferably Mo and q = 2, and in Formula (III), Z is preferably W.

The publication EP 2 765 136 A1 discloses a heteropolyoxometalate of the Formula (I), (II) or (III)

A_{q+3}PV_{q}Z_{12-q}O₄₀ (I),

A₆P₂Z₁₈O₆₂ (II),

or

A₃PZ₄O₂₄ (III)

wherein Z is selected from Mo or W,
q is 1, 2 or 3, and
A is selected among one or more cations and comprises at least one quaternary ammonium cation with the proviso that the compounds [(n-C₄H₉)₄N]₃PMo₁₂O₄₀ and [(n-C₆H₉)₄N]₃PMo₁₂O₄₀ are exempted. Most preferred are the heteropolyoxometalates [(n-C₄H₉)₄N]₃PW₄O₂₄ and [(n-C₆H₁₃)₄N]₃PW₄O₂₄.

Against this background it is an object of the present invention to provide a household appliance with improved hygiene. In particular, it is an object of the present invention to achieve an improved hygiene by applying an oxygen radicals containing aerosol as disinfectant.

In accordance with the present invention, this object is achieved by a household appliance and a method for operating a household appliance with the features of the independent claims. Preferred embodiments of the invention are detailed in the respective dependent claims. Preferred embodiments of the household appliance correspond to preferred embodiments of the method, even if they are not referred to herein in detail.

The invention is thus directed to a household appliance with a means to generate an airstream and an aerosol generator comprising at least one nebulizer and a liquid reservoir having at least one interior catalytically active surface containing a polyoxometalate, wherein the polyoxometalate has the Formula (I), (II) or (III),

A₃PZ₄O₂₄ (I)

A_{q+3}PV_{q}Z_{12-q}O₄₀ (II)

A₆P₂Z₁₈O₆₂ (III)

wherein Z is selected from Mo or W,
q is 0, 1, 2 or 3, and
A is selected from one or more cations selected from the group consisting of (XR₁R₂R₃R₄)⁺, wherein at least one of the groups R¹, R², R³, and R⁴ is a substituted or non-substituted alkyl, alkyloxoalkyl or cycloalkyl group that may contain at least one heteroatom selected among O, S, N or P, with at least five carbon atoms and wherein X is selected from among nitrogen and phosphorus.

Preferably, the polyoxometalate is a polyoxometalate of the Formula (I'), (II'), (III'), (IV') or (V'),

A₃PW₄O₂₄ (I'),

A_{q+3}PV_{q}MO_{12-q}O₄₀ (II),

A_{q+3}PV_{q}W_{12-q}O₄₀ (III'),

A₆P₂W₁₈O₆₂ (IV'),

or

A₆P₂MO₁₈O₆₂ (V')

wherein q is 0, 1 or 2.

In a more preferred embodiment, the polyoxometalate is

A₆P₂W₁₈O₆₂ (IV'),

or

A₆P₂Mo₁₈O₆₂ (V')

In an even more preferred embodiment, the polyoxometalate is

A_{q+3}PV_{q}Mo_{12-q}O₄₀ (II'),

or

A_{q+3}PV_{q}W_{12-q}O₄₀ (III'),

wherein q is 1 or 2.

In a particular preferred embodiment of the household appliance, the polyoxometalate is

A₃PW₄O₂₄ (I').

A household appliance is preferred, wherein the cation is selected from the group consisting of (NR¹R²R³R⁴)⁺, wherein at least one of the groups R¹, R², R³, and R⁴ is a substituted or nonsubstituted alkyl, alkyloxoalkyl or cycloalkyl group with at least eight carbon atoms.

In a preferred embodiment of the household appliance, the cation is selected from the group consisting of (PR¹R²R³R⁴)⁺, wherein at least one of the groups R¹, R², R³, and R⁴ is a substituted or nonsubstituted alkyl, alkyloxoalkyl or cycloalkyl group with at least eight carbon atoms.

It is moreover preferred that the polyoxometalate contains different cations. In this regard it is more preferably, that the polyoxometalate contains different cations of the formula (XR¹R²R³R⁴)⁺, wherein at least one of the groups R¹, R², R³, and R⁴ has at least ten carbon atoms. It is even more preferable that the cations have the formula (NR¹R²R³R⁴)⁺ and that the different cations have groups R¹, R², R³, or R⁴ with eight and ten carbon atoms. An advantageous mixture of cations is for example Aliquat 336 (Stark's catalyst) wherein the cations contain both octyl and decyl chains, i.e. alkyl chains with eight and ten carbon atoms, respectively.

In the household appliance of the present invention, the polyoxometalate may be used in the interior catalytically active surface of the liquid reservoir together with a polymeric organic or inorganic binder. Preferably, the binder is selected from among acrylate-based binders, polyurethane-based binders and silicone-based binders.

Suitable acrylate-based binders are for example Gräsolin 2K-Acryllack from the company Grasolin, WorléeCryl A1220 from the company Worlée Chemie, Bayhydrol® A145 from the company Covestro, a water-reducible, hydroxyfunctional polyacrylic dispersion, and Desmophen® A265BA from the company Covestro.

A suitable polyurethane (PUR)-based binder is for example ATCOAT Atrepur 340 from the company ATCOAT.

Suitable silicone resin-based binders are for example Silikopon® EF from the company Evonik, 2577 Low Voc from the company Dow Corning or Bluesil RES 991 from the company Bluesil.

In the production of a household appliance according to the invention, the interior catalytically active surface containing a polyoxometalate can be obtained for example by dip-coating the component into a liquid containing the binder and the polyoxometalate.

The household appliance is not limited. However, the household appliance is preferably a water-bearing household appliance. In general, a water-bearing household appliance is a household appliance during the operation of which water is used. The items to be cleaned can be, in particular, tableware or laundry items. Cleaning should according to the invention also be understood to mean freshening. Accordingly, a water-bearing household appliance can be also a dryer.

In a preferred embodiment, the water-bearing household appliance is a dishwasher or a laundry treatment device or a coffee making machine.

In another preferred embodiment, the water-bearing household appliance is belonging to the group consisting of a washing machine, a washer-dryer and a dryer.

In the present invention, the property of the polyoxometalates, and in particular of the specific polyoxometalates as described herein which do not need an activation by radiation, as oxidation catalyst or as oxidation agent is utilized. Generally, the effects of the polyoxometalate can be two-fold, the extent of these effects being dependent on the specific polyoxometalate used.

For example, a cause of disinfection might be an oxidation of organic substances by the polyoxometalate itself when they are in direct contact. Then the polyoxometalate would be itself reduced. Such an oxidation may lead to the partial or complete transformation of the microorganisms, depending on the extent of oxidation. Sometimes even a minor oxidative impact on the microorganism might be sufficient, if the microorganism can be transformed into a state wherein reproduction is no longer possible.

More importantly, however, the polyoxometalate containing surface is catalytically effective in oxidation reactions. In the oxidation reaction, the polyoxometalate serves, in general, as an oxidation catalyst which cooperates with an oxidizing agent. The oxidizing agent is not limited according to the invention. Preferably, an oxygen-containing oxidizing agent is used. Oxygen, inorganic or organic peroxides and/or ozone are particularly preferred as oxidizing agents. Oxygen is again particularly preferred amongst them as an oxidizing agent since an additional input of possibly harmful or interfering substances can be avoided. Air is preferably used as a source of oxygen. However, the oxygen may be present, for example, dissolved in water. Furthermore, by means of a corresponding movement of the water, mixing of air and water can take place, for example, for the formation of air bubbles in the water and an air-water mixture of this type can be brought into contact with the polyoxometalate-containing surface, the atmospheric oxygen preferably serving as the oxidizing agent.

Thus, in a preferred embodiment the liquid reservoir of the aerosol generator comprises furthermore an air infeed element. Preferably, the air infeed element is connected to the liquid reservoir at a position below a predetermined filling level of aqueous liquid to provide a sufficient quantity of oxygen in the aqueous liquid.

If the household appliance is a washer-dryer or a washing machine and the aqueous liquid inside the liquid reservoir of the aerosol generator is stored water, for example greywater, a peroxide may advantageously be used as the oxidizing agent as peroxide is generally present in laundry care products used.

In the presence of an oxidizing agent such as oxygen, hydrogen peroxide or ozone, oxygen radicals (or oxygen-containing radicals, herein referred altogether as "oxygen radicals") form at the interior polyoxometalate-containing surface in the liquid reservoir of the aerosol generator. If an aqueous liquid thus comes into contact with the at least one interior polyoxometalate-containing surface, it is thus enriched with oxygen radicals.

Preferably, the liquid reservoir thus has two interior polyoxometalate-containing surfaces. It is even more preferable, if all interior surfaces in the liquid reservoir are catalytically active containing a polyoxometalate.

When these highly reactive oxygen radicals come into contact with organic substances including microorganisms, an oxidation reaction takes place which leads to the decomposition of the organic substances and/or to pathological processes in the microorganisms. In this way, the formation of a biofilm is counteracted.

In order to enable a continuous oxidation reaction inside the liquid reservoir, a sufficient level of mobility of the oxidizing species, for example, of oxygen radicals which have been generated at the polyoxometalate-containing surface acting as an oxidation catalyst is preferable. This could be for example achieved by recirculating the aqueous liquid inside the liquid reservoir with the aid of a pump. It might also be sufficient to exploit natural diffusion, or convection in case of temperature differences. Said temperature differences can occur for example in washer-dryers or dryers if the liquid reservoir of the aerosol generator is located near an outer wall of the housing of said washer-dryer or dryer. Generally, due to the heating of process air, the temperature towards the inner part of the washer-dryer or dryer is higher than the temperature near the outer wall of the housing, which creates a temperature gradient inside the liquid reservoir thus inducing natural convection in the aqueous liquid.

Furthermore, a sufficiently high concentration of oxidizing agent should be present in the aerosol in order to achieve a sufficient hygienic effect.

In the household appliance of the present invention it is preferred that the interior catalytically active surface in the liquid reservoir has a polyoxometalate containing upper layer with a thickness of from 0.01 to 1.5 mm, preferably a thickness of from 0.01 to 0.5 mm, more preferably of from 0.02 to 1 mm, and even more preferably of from 0.05 to 0.5 mm.

When the polyoxometalate is used together with an acrylate-based binder, a polyurethane-based binder or a silicone resin-based binder, the coating thickness is preferably in the range of from 10 nm to 500 µm and more preferably in the range of from 20 nm to 200 µm.

It is moreover preferred that the upper layer of the surface has a polyoxometalate content in the range of from 0.5 to 50 wt%, more preferably in the range of from 1 to 15 wt%, and even more preferably in the range of from 1 to 10 wt%, based on the weight of the upper layer (in the following also to be termed "surface layer").

The interior catalytically active surface containing polyoxometalate can be created in any way, provided the catalytic effect according to the invention is possible. For example, said surface can be created by forming a polyoxometalate-containing film or, for example, by placing polyoxometalate particles at the surface of a porous material using in both cases a suitable binder.

In order to maintain the polyoxometalate at the surface and to prevent dissolution into a surrounding liquid medium or any other depletion because of the contact with a surrounding medium, it is important to use the specific cations described herein. Organic ions with a long chain are especially suitable as described herein. Particularly preferred examples thereof are the methyltrioctylammonium and tributyltetradecylphosphonium cations.

It is noted that an additional antimicrobial effect is achieved by the use of the cations described herein.

The polyoxometalate described herein is a salt which can be bound by means of suitable binder systems. In addition, the polyoxometalate may be admixed with the remainder of the material in the surface, ahead of the manufacture, for example an organic polymeric material comprising a polyamide or a polyalkylene, for example polypropylene.

Moreover, it is possible to produce a foil wherein the polyoxometalate is combined with a suitable carrier material and the binder, such that only a comparatively small amount of polyoxometalate might be required in order to cover a large surface area. Such foils can be applied for example by glueing or meltbonding. Moreover such foils may be placed directly in an injection mould and thus directly utilized for the manufacture.

In the present invention, the property of the polyoxometalates, and in particular of the specific polyoxometalates as described herein which do not need an activation by radiation, as oxidation catalyst or as oxidation agent is utilized in the form of an aerosol. According to the invention the aerosol is created in an aerosol generator comprising a nebulizer and a liquid reservoir having at least one interior catalytically active surface containing a polyoxometalate.

An aerosol is generally a dispersion of solid and/or liquid particles in a gas. Preferably, in the present invention the gas is air and the particles are of an aqueous liquid. The term "aqueous liquid" is to be understood as a water-based liquid that may also be just water. The aqueous liquid may further not only be based on fresh water, but also on water stored in a household appliance, as for example greywater in a washing machine.

As already stated above, the aqueous liquid inside the liquid reservoir is enriched with oxygen radicals due to contact with the at least one catalytically active surface containing polyoxometalate. If an aerosol is created from said aqueous liquid by nebulizing the liquid, the aerosol will also be enriched with said oxygen radicals as the liquid particles will contain the oxygen radicals as well.

In this context it is noted that cold nebulization is preferred. At high temperatures the oxygen radicals might be destroyed during the nebulization step or their lifetime might be reduced, which is disadvantageous if an effective disinfection is to be achieved.

In order to create a sufficient concentration of aerosol according to the present invention, the liquid reservoir exhibits a predetermined filling-level for the aqueous liquid. In this way, it is ensured that in the liquid reservoir a volume of space above the surface of the aqueous liquid is left free. Aerosol that is created by nebulizing the aqueous liquid inside the liquid reservoir may then concentrate in this free volume of space.

Preferably if the aqueous liquid is fresh water, the liquid reservoir may be connected to a fresh water supply. In a preferred embodiment the fresh water supply may then be opened and closed by a valve operable by a control system. In an even more preferred embodiment a relation between the volume of fresh water V_{fw} filled into the liquid reservoir and the opening time tₒₚₑₙ of the valve is stored in the control system so as to not exceed the predetermined filling-level.

Preferably if the aqueous liquid is stored water, the liquid reservoir may be connected to a pump operable by a control system. In a water-bearing household appliance said pump may be for example a lye pump or a pump for condensed water. In an even more preferred embodiment, a relationship between the operating time tₒₚₑᵣₐₜₑ of the pump and the volume of the stored water V_{sw} filled into the liquid reservoir is stored in the control system so as to not exceed the predetermined filling-level.

In another preferred embodiment, the liquid reservoir further comprises a filling-level-sensor placed at the predetermined filling-level. Once the filling-level-sensor comes into contact with aqueous liquid a signal response is generated and sent to a control system. Depending on how water is filled into the liquid reservoir the control system may then for example close a valve to the fresh water supply or stop a pump operation.

Preferably, the liquid reservoir may also comprise an overfill protection to avoid exceeding the predetermined filling-level. Such an overfill protection may be a valve that automatically opens once a certain pressure level is reached or a valve that can be operated by a control system once a signal response is given by a filling-level-sensor placed at the predetermined filling-level.

According to the present invention the aerosol in the aerosol generator is created by at least one nebulizer.

The type of nebulizer used to create the aerosol is not limited. Examples for a nebulizer include a spray nozzle, a rotary nozzle or an ultrasonic nebulizer. In a preferred embodiment, however, the nebulizer is an ultrasonic nebulizer.

The arrangement of the nebulizer inside the liquid reservoir is also not limited and will surely depend on the type of nebulizer used and the specific conditions inside the liquid reservoir.

In a preferred embodiment, the nebulizer is an ultrasound nebulizer comprising at least one excitation area that is arranged as such that the excitation area forms at least a part of the bottom of the liquid reservoir.

In another preferred embodiment, the nebulizer is an ultrasound nebulizer comprising at least one excitation area that is arranged as such that the excitation area is positioned at the predetermined filling-level pointing in downward direction, i.e. towards the bottom of the liquid reservoir.

If an ultrasound nebulizer is provided such that its excitation area is positioned at the predetermined filling-level, the liquid reservoir may also comprise a separating wall at the predetermined filling-level. The separating wall may then serve to separate the area wherein the ultrasound nebulizer is placed in from the area wherein the aerosol is created in. In this way, the aerosol concentration above the surface of the aqueous liquid can be increased.

If more than one nebulizer is used, the nebulizers may be located at different positions inside the liquid reservoir. Preferably one ultrasound nebulizer is then arranged at the predetermined filling-level inside the liquid reservoir, whereas the second ultrasound nebulizer is then arranged at the bottom of the liquid reservoir. Even more preferably, the bottom of the liquid reservoir is then at least partially formed by the excitation area of the second ultrasound nebulizer.

The at least one nebulizer is adapted to nebulize at least a part of the aqueous liquid inside the liquid reservoir. The amount of aqueous liquid nebulized is generally not limited, but will surely depend on the concentration of aerosol required for disinfection of the respective household appliance. In a preferred embodiment, the nebulizer is thus operable by a control system. Preferably, a relation between the operating time and power of the nebulizer and the concentration of aerosol created in the aerosol generator is then stored in the control system.

In general, the size and shape of the liquid reservoir of the aerosol generator is not limited. Size and shape will surely depend on the available construction space, the requirements of the household appliance and/or on the type of nebulizer used. The liquid reservoir may thus exhibit for example a cubic or rectangular shape, but may also have a more complex shape.

However, it is noted that the inner diameter of the liquid reservoir is preferably largest at the predetermined filling-level.

In a preferred embodiment the liquid reservoir has in at least one section a monotonic decreasing inner diameter from the predetermined filling-level in downward direction. Preferably, the inner diameter is strictly monotonic decreasing. Even more preferably, the inner diameter of the liquid reservoir decreases steadily, as well.

In a preferred embodiment the shape of the at least one section in which the inner diameter of the liquid reservoir is monotonic decreasing has a shape selected from the group consisting of conical, concave, convex and ellipsoidal.

If the shape is conical and an ultrasound nebulizer is provided at the bottom of the liquid reservoir, the propagation of the ultrasound waves up to the surface of the aqueous liquid inside the reservoir is improved and thus aerosol can be created more effectively above the surface of the aqueous liquid.

If the shape is convex and an ultrasound nebulizer is provided at the bottom of the liquid reservoir, a lobe-like intensity distribution of the ultrasonic waves can be achieved improving the effectiveness of aerosol formation at the surface of the aqueous liquid.

If the shape is concave and an ultrasound nebulizer is provided as such that its excitation area is positioned at the predetermined filling-level pointing in downward direction a collimation of the ultrasound waves may be achieved in the concave section. The effectiveness of aerosol formation may then be adjusted via the curvature of the surface in the concave section.

If the shape is ellipsoidal and an ultrasound nebulizer is provided as such that its excitation area is positioned at the predetermined filling-level pointing in downward direction, the mode of operation of a concave mirror with respect to the propagation of ultrasound waves can be exploited to improve the effectiveness of aerosol formation.

According to the invention, the household appliance also has a means to generate an airstream. The airstream serves to direct the aerosol to the surfaces inside the household appliance that shall be disinfected. Preferably, thus a channel is connected to the part of the liquid reservoir where the aerosol is created in. The distribution of the aerosol in the household appliance may generally be achieved by using pipes, hoses and/or baffle plates, but other methods are also conceivable.

The means to generate the airstream is not limited. All means capable of generating said airstream may be used, for example a pump, a ventilator or else. The means to generate the airstream may be already present in the household appliance and used for other purposes. Preferably, the means may then be adapted to additionally direct the aerosol to the surfaces that shall be disinfected. The means to generate an airstream may also be built in separately and used to distribute the aerosol only. This may be the case for household appliances such as washing machines or coffee machines that generally do not comprise a means to generate an airstream.

In a preferred embodiment, the household appliance is a washer-dryer or a dryer comprising a process air fan. Preferably, the process air fan is then additionally adapted to generate an airstream to direct the aerosol created in the aerosol generator to the surfaces inside the washer-dryer or dryer that shall be disinfected.

If the means to generate an airstream is a process air fan, preferably, the liquid reservoir of the aerosol generator is connected to the process air channel, for example via a bypass. Even more preferably, then a switch is provided to select whether the airstream generated by the process air fan flows through the process air channel or the bypass channel.

The invention is moreover directed to a method for operating a household appliance with a means to generate an airstream and an aerosol generator comprising at least one nebulizer and a liquid reservoir having at least one interior catalytically active surface containing a polyoxometalate, wherein the polyoxometalate has the Formula (I), (II) or (III),

A₃PZ₄O₂₄ (I)

A_{q+3}PV_{q}Z_{12-q}O₄₀ (II)

A₆P₂Z₁₈O₆₂ (III)

wherein Z is selected from Mo or W,
q is 0, 1, 2 or 3, and
A is selected from one or more cations selected from the group consisting of (XR¹R²R³R⁴)⁺, wherein at least one of the groups R¹, R², R³, and R⁴ is a substituted or non-substituted alkyl, alkyloxoalkyl or cycloalkyl group that may contain at least one heteroatom selected among O, S, N or P, with at least five carbon atoms and wherein X is selected from among nitrogen and phosphorus, wherein it comprises the following steps:
   (a) filling the liquid reservoir with an aqueous liquid up to a predetermined filling-level to bring the aqueous liquid into contact with the at least one interior catalytically active surface containing a polyoxometalate;
   (b) nebulizing at least a part of the aqueous liquid inside the liquid reservoir with the at least one nebulizer so as to create an aerosol in the liquid reservoir above the predetermined filling-level; and
   (c) generating an airstream so as to direct the aerosol formed in step b) to surfaces in the household appliance.

The invention has the advantage that a household appliance with improved hygiene is made available in a simple and cost-effective manner. In particular, this is advantageous in the case of a water-bearing household appliance which has a markedly lower susceptibility to dirt especially involving microorganisms.

A particular advantage of the use of the polyoxometalates described herein lies in the fact that polyoxometalates are able to function for years without additional activation or energy input as a catalytic system. Thus, the oxygen radicals, which disinfect the interior of the household appliance, can be produced easily and continuously. The simple way to distribute the oxygen radicals in the form of an aerosol is also a very energy efficient solution compared to methods such as hot steam treatment. Moreover, the aerosol can be used to easily reach all relevant surfaces within the appliance, especially undercuts or corners are difficult to reach with conventional methods. The invention is furthermore also eco-friendly and easy to use as no refill of harmful chemical substances is necessary and no manual handling is involved.

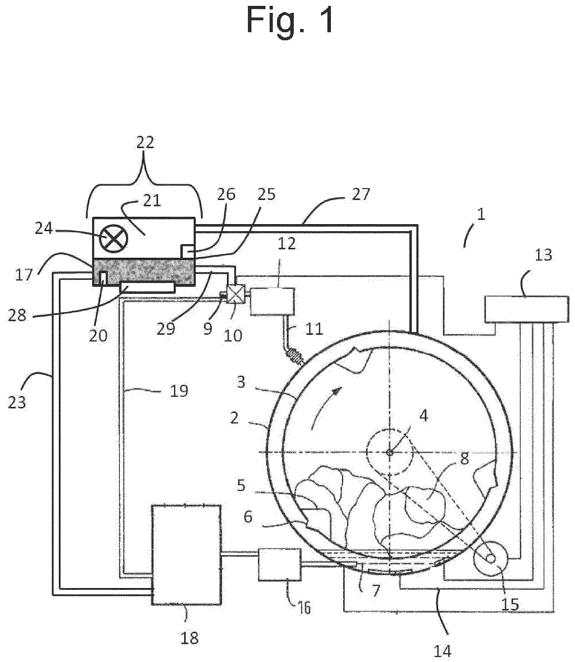

In the following,a preferred embodiment of the invention will be described in more detail by reference to Fig.1 of the drawing attached.

Fig. 1 is a schematic representation of the present relevant parts of an embodiment of the household appliance according to the invention configured as a washing machine as non-limiting example.

The washing machine 1 in this embodiment has an outer tub 2 in which a drum 3 is rotatably mounted and can be driven by a drive motor 15. For ergonomic reasons, the rotation axis 4 of the drum 3 is oriented upwardly out of the horizontal by a small angle, so that easier access to, and inspection of the interior of the drum 3 is provided. With this arrangement, in cooperation with specially formed laundry agitators 5 and scooping devices 6 for the washing liquor 7 at the internal surface of the drum jacket, intensification of the flow of washing liquor 7 through the laundry items 8 can be achieved.

The washing machine also has a water feed system which comprises a water connection fixture for the domestic water supply 9, an electrically controllable valve 10 and a feed pipe 11 which extends to the outer tub 2 and is fed via a detergent dispenser tray 12 from which the feed liquid is able to transport washing agent portions to the outer tub 2.

A heating device 14 is also provided in the outer tub 2. The valve 10 and the heating device 14 can be controlled by a program control system 13 depending on a program execution plan which can be linked to a time program and/or to the reaching of particular measured values of parameters such as the liquor level, the liquor temperature, the rotary speed of the drum 3 etc. within the washing machine 1. 16 denotes a pump for the liquid in particular washing liquor 7 or suds 7, in the outer tub 2.

A water reservoir 18 can store the greywater which has been used for rinsing the laundry item 8. Said greywater can be used for a later washing cycle or for filling the liquid reservoir 21 of the aerosol generator 22. For this purpose the water reservoir 18 is connected via a line ("feed pipe for rinsing fluid") 19 to the valve 10 which also regulates the fresh water feed. The water reservoir 18 is furthermore connected via a second line 23 to the liquid reservoir 21 of the aerosol generator 22. The valve 10 is a three way valve to either provide fresh water supply for the water reservoir 18 or the liquid reservoir 21 via supply line 29. An air infeed element 20 which can also be controlled by the program control system 13 is provided in the liquid reservoir 21. With these measures a particularly efficient oxidation reaction is possible at the interior surface 17 in the liquid reservoir 21 of the aerosol generator 22.

The interior surface 17 of the liquid reservoir 21 contains A₃PWO₂₄, wherein A is either the cation methyltrioctylammonium or the cation tributyltetradecylphosphonium, or a mixture thereof.

The aerosol generator 22 is furthermore provided with a ventilator 24, which is positioned in the upper part of the liquid reservoir 21 above the predetermined filling-level 25 and can be controlled by the control system 13. A filling-level-sensor 26 is provided to respond to the control system 13 when the predetermined filling-level 25 is reached. Above the predetermined filling-level 25 the liquid reservoir 21 is furthermore connected to a channel 27 through which the aerosol can be directed to the relevant surfaces in the washing machine 1 that shall be disinfected. The aerosol generator 22 is also provided with an ultrasound nebulizer 28 at the bottom of the liquid reservoir 21. The excitation area of the ultrasound nebulizer 28 forms a part of the bottom of the liquid reservoir 21.

The ultrasound nebulizer 28 serves to nebulize at least a part of the aqueous liquid inside the liquid reservoir 21. The aerosol thus created concentrates above the surface of the aqueous liquid, i.e. in the part of the liquid reservoir 21 which is above the predetermined filling-level 25. If the aerosol concentration is sufficiently high the ventilator 24 generates an airstream that directs the aerosol via the channel 27 to the relevant surfaces in the washing machine 1 that shall be disinfected. Preferably, said distribution of aerosol is realized via pipes, hoses and baffle plates which are not shown in Fig. 1 for reasons of clarity. The concentration of aerosol formed is furthermore preferably determined by the operating power and time of the ultrasound nebulizer 28 based on a calibration curve stored in the control system 13.

### REFERENCE NUMERALS

- 1: Household appliance, water-bearing household appliance, washing machine
- 2: (Outer) tub
- 3: Laundry drum
- 4: Rotation axis of the drum
- 5: Laundry agitators
- 6: Scooping devices
- 7: Washing liquor
- 8: Laundry items
- 9: Domestic water supply
- 10: Valve
- 11: Feed pipe
- 12: Detergent dispenser tray
- 13: Program control system
- 14: Heating device
- 15: Drive motor
- 16: Pump
- 17: Interior surface containing polyoxometalate
- 18: Water reservoir
- 19: Feed pipe for rinsing fluid
- 20: Air infeed element
- 21: Liquid reservoir (of the aerosol generator)
- 22: Aerosol generator
- 23: Connecting line
- 24: Ventilator
- 25: Predetermined filling-level
- 26: Filling-level-sensor
- 27: Channel
- 28: Nebulizer, ultrasound nebulizer
- 29: Fresh water supply line

## Claims

1. Household appliance (1) with a means to generate an airstream (24) and an aerosol generator (22) comprising at least one nebulizer (28) and a liquid reservoir (21) having at least one interior catalytically active surface containing a polyoxometalate (17), **characterized in that** the polyoxometalate has the Formula (I), (II) or (III),
A₃PZ₄O₂₄ (I)
A_{q+3}PV_{q}Z_{12-q}O₄₀ (II)
A₆P₂Z₁₈O₆₂ (III)
wherein Z is selected from Mo or W,
q is 0, 1, 2 or 3, and
A is selected from one or more cations selected from the group consisting of (XR¹R²R³R⁴)⁺, wherein at least one of the groups R¹, R², R³, and R⁴ is a substituted or non-substituted alkyl, alkyloxoalkyl or cycloalkyl group that may contain at least one heteroatom selected among O, S, N or P, with at least five carbon atoms and wherein X is selected from among nitrogen and phosphorus.

2. The household appliance (1) according to claim 1, wherein the polyoxometalate is
A₃PW₄O₂₄ (I')

3. The household appliance (1) according to claim 1 or 2, wherein the cation is selected from the group consisting of (NR¹R²R³R⁴)⁺, wherein at least one of the groups R¹, R², R³, and R⁴ is a substituted or non-substituted alkyl, alkyloxoalkyl or cycloalkyl group with at least eight carbon atoms.

4. The household appliance (1) according to any of claims 1 to 3, wherein the cation is selected from the group consisting of (PR¹R²R³R⁴)⁺, wherein at least one of the groups R¹, R², R³, and R⁴ is a substituted or non-substituted alkyl, alkyloxoalkyl or cycloalkyl group with at least eight carbon atoms.

5. The household appliance (1) according to any of claims 1 to 4, wherein the polyoxometalate contains different cations.

6. The household appliance (1) according to any of claims 1 to 5, wherein the polyoxometalate contains different cations of the formula (XR¹R²R³R⁴)⁺, wherein at least one of the groups R¹, R², R³, and R⁴ has at least ten carbon atoms.

7. The household appliance (1) according to claim 6, wherein the cations have the formula (NR¹R²R³R⁴)⁺ and wherein the different cations have groups R¹, R², R³, or R⁴ with eight and ten carbon atoms.

8. The household appliance (1) according to any of claims 1 to 7, wherein the liquid reservoir (21) in at least one section has a monotonic decreasing inner diameter from a predetermined filling-level (25) in downward direction.

9. The household appliance (1) according to claim 8, wherein the liquid reservoir (21) in the at least one section has a shape selected from the group consisting of conical, concave, convex and ellipsoidal.

10. The household appliance (1) according to any of claims 1 to 9, wherein the at least one nebulizer (28) is an ultrasound nebulizer comprising at least one excitation area, wherein the excitation area forms at least a part of the bottom of the liquid reservoir (21).

11. The household appliance (1) according to any of claims 1 to 9, wherein the at least one nebulizer (28) is an ultrasound nebulizer comprising at least one excitation area, wherein the excitation area is positioned at a predetermined filling-level (25) inside the liquid reservoir (21) pointing in downward direction.

12. The household appliance (1) according to any of claims 1 to 11, **characterized in that** the household appliance is a washer-dryer or a dryer and that the means to generate an airstream (24) is a process air fan.

13. The household appliance (1) according to any of claims 1 to 12, wherein the liquid reservoir (21) further comprises a filling-level-sensor (26), wherein the filling-level-sensor (26) is placed at a predetermined filling-level (25) inside the liquid reservoir (21).

14. The household appliance (1) according to any of claims 1 to 13, wherein the liquid reservoir (21) further comprises an air infeed element (20).

15. Method for operating a household appliance (1) with a means to generate an airstream (24) and an aerosol generator (22) comprising at least one nebulizer (28) and a liquid reservoir (21) having at least one interior catalytically active surface containing a polyoxometalate (17), wherein the polyoxometalate has the Formula (I), (II) or (III),
A₃PZ₄O₂₄ (I)
A_{q+a}PV_{q}Z_{12-q}O₄₀ (II)
A₆P₂Z₁₈O₆₂ (III)
wherein Z is selected from Mo or W,
q is 0, 1, 2 or 3, and
A is selected from one or more cations selected from the group consisting of (XR¹R²R³R⁴)⁺, wherein at least one of the groups R¹, R², R³, and R⁴ is a substituted or non-substituted alkyl, alkyloxoalkyl or cycloalkyl group that may contain at least one heteroatom selected among O, S, N or P, with at least five carbon atoms and wherein X is selected from among nitrogen and phosphorus, **characterized in that** it comprises the following steps:
(a) filling the liquid reservoir (21) with an aqueous liquid up to a predetermined filling-level (25) to bring the aqueous liquid into contact with the at least one interior catalytically active surface containing a polyoxometalate (17);
(b) nebulizing at least a part of the aqueous liquid inside the liquid reservoir (21) with the at least one nebulizer (28) so as to create an aerosol in the liquid reservoir (21) above the predetermined filling-level (25); and
(c) generating an airstream so as to direct the aerosol formed in step (b) to surfaces in the household appliance (1).

## Patentansprüche

1. Haushaltsgerät (1) mit einem Mittel zum Erzeugen eines Luftstroms (24) und einem Aerosolgenerator (22), der wenigstens einen Zerstäuber (28) und einen Flüssigkeitsbehälter (21) umfasst, der wenigstens eine katalytisch aktive Innenfläche umfasst, die ein Polyoxometalat (17) enthält, **dadurch gekennzeichnet, dass** das Polyoxometalat die Formel (I), (II) oder (III) hat,
A₃PZ₄O₂₄ (I)
A_{q+3}PV_{q}Z_{12-q}O₄₀ (II)
A₆P₂Z₁₈O₆₂ (III)
wobei Z aus Mo oder W ausgewählt ist
q 0, 1, 2 oder 3 ist und
A aus einem oder mehr aus der Gruppe bestehend aus (XR¹R²R³R⁴)⁺ ausgewählten Kationen ausgewählt ist, wobei wenigstens eine der Gruppen R¹, R², R³ und R⁴ eine substituierte oder unsubstituierte Alkyl-, Alkyloxoalkyl- oder Cycloalkyl-Gruppe, die wenigstens ein aus O, S, N oder P ausgewähltes Heteroatom enthalten kann, mit wenigstens fünf Kohlenstoffatomen ist, und wobei X aus Stickstoff und Phosphor ausgewählt ist.

2. Haushaltsgerät (1) nach Anspruch 1, wobei das Polyoxometalat
A₃PW₄O₂₄ (I')
ist.

3. Haushaltsgerät (1) nach Anspruch 1 oder 2, wobei das Kation aus der Gruppe bestehend aus (NR¹R²R³R⁴)⁺ ausgewählt ist, wobei wenigstens eine der Gruppen R¹, R², R³ und R⁴ eine substituierte oder unsubstituierte Alkyl-, Alkyloxoalkyl- oder Cycloalkyl-Gruppe mit wenigstens acht Kohlenstoffatomen ist.

4. Haushaltsgerät (1) nach einem der Ansprüche 1 bis 3, wobei das Kation aus der Gruppe bestehend aus (PR¹R²R³R⁴)⁺ ausgewählt ist, wobei wenigstens eine der Gruppen R¹, R², R³ und R⁴ eine substituierte oder unsubstituierte Alkyl-, Alkyloxoalkyl- oder Cycloalkyl-Gruppe mit wenigstens acht Kohlenstoffatomen ist.

5. Haushaltsgerät (1) nach einem der Ansprüche 1 bis 4, wobei das Polyoxometalat verschiedene Kationen enthält.

6. Haushaltsgerät (1) nach einem der Ansprüche 1 bis 5, wobei das Polyoxometalat verschiedene Kationen der Formel (XR¹R²R³R⁴) enthält, wobei wenigstens eine der Gruppen R¹, R², R³ und R⁴ wenigstens 10 Kohlenstoffatome aufweist.

7. Haushaltsgerät (1) nach Anspruch 6, wobei die Kationen die Formel (NR¹R²R³R⁴)⁺ haben und wobei die unterschiedlichen Kationen Gruppen R¹, R², R³ oder R⁴ mit acht und zehn Kohlenstoffatomen aufweisen.

8. Haushaltsgerät (1) nach einem der Ansprüche 1 bis 7, wobei der Flüssigkeitsbehälter (21) in wenigstens einem Abschnitt einen monotonisch abnehmenden Innendurchmesser von einem vorbestimmten Füllstand (25) in Abwärtsrichtung hat.

9. Haushaltsgerät (1) nach Anspruch 8, wobei der Flüssigkeitsbehälter (21) in dem wenigstens einen Abschnitt eine Form hat, die aus der Gruppe bestehend aus konisch, konkav, konvex und elliptisch ausgewählt ist.

10. Haushaltsgerät (1) nach einem der Ansprüche 1 bis 9, wobei der wenigstens eine Zerstäuber (28) ein Ultraschallzerstäuber ist, der wenigstens eine Anregungsfläche umfasst, wobei die Anregungsfläche wenigstens ein Teil des Bodens des Flüssigkeitsbehälters (21) ist.

11. Haushaltsgerät (1) nach einem der Ansprüche 1 bis 9, wobei der wenigstens eine Zerstäuber (28) ein Ultraschallzerstäuber ist, der wenigstens eine Anregungsfläche umfasst, wobei die Anregungsfläche an einem vorbestimmten Füllstand (25) innerhalb des Flüssigkeitsbehälters (21) nach unten weisend angeordnet ist.

12. Haushaltsgerät (1) nach einem der Ansprüche 1 bis 11, **dadurch gekennzeichnet, dass** das Haushaltsgerät ein Waschtrockner oder ein Trockner ist und dass das Mittel zum Erzeugen eines Luftstroms (24) ein Prozessluftgebläse ist.

13. Haushaltsgerät (1) nach einem der Ansprüche 1 bis 12, wobei der Flüssigkeitsbehälter (21) ferner einen Füllstandsensor (26) umfasst, wobei der Füllstandsensor (26) an einem vorbestimmten Füllstand (25) innerhalb des Flüssigkeitsbehälters (21) angeordnet ist.

14. Haushaltsgerät (1) nach einem der Ansprüche 1 bis 13, wobei der Flüssigkeitsbehälter (21) ferner ein Lufteinzugselement (20) umfasst.

15. Verfahren zum Betreiben eines Haushaltsgeräts (1) mit einem Mittel zum Erzeugen eines Luftstroms (24) und einem Aerosolgenerator (22), der wenigstens einen Zerstäuber (28) und einen Flüssigkeitsbehälter (21) umfasst, der wenigstens eine katalytisch aktive Innenfläche umfasst, die ein Polyoxometalat (17) enthält, wobei das Polyoxometalat die Formel (I), (II) oder (III) hat,
A₃PZ₄O₂₄ (I)
A_{q+3}PV_{q}Z_{12-q}O₄₀ (II)
A₆P₂Z₁₈O₆₂ (III)
wobei Z aus Mo oder W ausgewählt ist
q 0, 1, 2 oder 3 ist und
A aus einem oder mehr aus der Gruppe bestehend aus (XR¹R²R³R⁴)⁺ ausgewählten Kationen ausgewählt ist, wobei wenigstens eine der Gruppen R¹, R², R³ und R⁴ eine substituierte oder unsubstituierte Alkyl-, Alkyloxoalkyl- oder Cycloalkyl-Gruppe, die wenigstens ein aus O, S, N oder P ausgewähltes Heteroatom enthalten kann, mit wenigstens fünf Kohlenstoffatomen ist, und wobei X aus Stickstoff und Phosphor ausgewählt ist, **dadurch gekennzeichnet, dass** es die folgenden Schritte umfasst:
(a) Füllen des Flüssigkeitsbehälters (21) mit einer wässrigen Flüssigkeit bis zu einem vorbestimmten Füllstand (25), um die wässrige Flüssigkeit mit der wenigstens einen katalytisch aktiven Innenfläche, die ein Polyoxometalat (17) enthält, in Berührung zu bringen,
(b) Zerstäuben von wenigstens einem Teil der wässrigen Flüssigkeit innerhalb des Flüssigkeitsbehälters (21) mit dem wenigstens einen Zerstäuber (28), um somit ein Aerosol in dem Flüssigkeitsbehälter (21) oberhalb des vorbestimmten Füllstands (25) zu erzeugen, und
(c) Erzeugen eines Luftstroms, um somit das in Schritt (b) gebildete Aerosol in das Haushaltsgerät (1) zu leiten.

## Revendications

1. Appareil ménager (1) avec un moyen pour générer un courant d'air (24) et un générateur d'aérosol (22) comprenant au moins un nébuliseur (28) et un réservoir de liquide (21) ayant au moins une surface intérieure active par catalyse contenant un polyoxométallate (17), **caractérisé en ce que** le polyoxométallate possède la Formule (I), (II) ou (III),
A₃PZ₄O₂₄ (1)
A_{q+3}PV_{q}Z_{12-q}O₄₀ (II)
A₆P₂Z₁₈O₆₂ (III)
dans lequel Z est sélectionné parmi Mo ou W,
q est égal à 0, 1, 2 ou 3, et
A est choisi parmi un ou plusieurs cations sélectionnés parmi le groupe constitué par (XR¹R²R³R⁴)⁺, dans lequel au moins l'un des groupes R¹, R², R³ et R⁴ est un groupe alkyle, alkyloxoalkyle ou cycloalkyle substitué ou non substitué qui peut contenir au moins un hétéroatome sélectionné parmi O, S, N ou P, ayant au moins cinq atomes de carbone et dans lequel X est sélectionné parmi l'azote et le phosphore.

2. Appareil ménager (1) selon la revendication 1, dans lequel le polyoxométallate est
A₃PW₄O₂₄ (I')

3. Appareil ménager (1) selon la revendication 1 ou 2, dans lequel le cation est sélectionné parmi le groupe constitué par (NR¹R²R³R⁴)⁺, dans lequel au moins l'un des groupes R¹, R², R³ et R⁴ est un groupe alkyle, alkyloxoalkyle ou cycloalkyle substitué ou non substitué ayant au moins huit atomes de carbone.

4. Appareil ménager (1) selon l'une quelconque des revendications 1 à 3, dans lequel le cation est sélectionné parmi le groupe constitué par (PR¹R²R³R⁴)⁺, dans lequel au moins l'un des groupes R¹, R², R³ et R⁴ est un groupe alkyle, alkyloxoalkyle ou cycloalkyle substitué ou non substitué ayant au moins huit atomes de carbone.

5. Appareil ménager (1) selon l'une quelconque des revendications 1 à 4, dans lequel le polyoxométallate contient différents cations.

6. Appareil ménager (1) selon l'une quelconque des revendications 1 à 5, dans lequel le polyoxométallate contient différents cations de la formule (XR¹R²R³R⁴)⁺, dans lequel au moins l'un des groupes R¹, R², R³ et R⁴ possède au moins dix atomes de carbone.

7. Appareil ménager (1) selon la revendication 6, dans lequel les cations possèdent la formule (NR¹R²R³R⁴)⁺ et dans lequel les différents cations possèdent des groupes R¹, R², R³ ou R⁴ ayant huit et dix atomes de carbone.

8. Appareil ménager (1) selon l'une quelconque des revendications 1 à 7, dans lequel le réservoir de liquide (21) dans au moins une section possède un diamètre interne décroissant de façon monotone à partir d'un niveau de remplissage prédéterminé (25) dans une direction orientée vers le bas.

9. Appareil ménager (1) selon la revendication 8, dans lequel le réservoir de liquide (21) dans l'au moins une section possède une forme sélectionnée parmi le groupe constitué par les formes conique, concave, convexe et ellipsoïdale.

10. Appareil ménager (1) selon l'une quelconque des revendications 1 à 9, dans lequel l'au moins un nébuliseur (28) est un nébuliseur ultrasonore comprenant au moins une zone d'excitation, dans lequel la zone d'excitation forme au moins une partie du fond du réservoir de liquide (21).

11. Appareil ménager (1) selon l'une quelconque des revendications 1 à 9, dans lequel l'au moins un nébuliseur (28) est un nébuliseur ultrasonore comprenant au moins une zone d'excitation, dans lequel la zone d'excitation est positionnée à un niveau de remplissage prédéterminé (25) à l'intérieur du réservoir de liquide (21) dirigé dans une direction orientée vers le bas.

12. Appareil ménager (1) selon l'une quelconque des revendications 1 à 11, **caractérisé en ce que** l'appareil ménager est un lave-linge séchant ou un sèche-linge et que le moyen pour générer un courant d'air (24) est un ventilateur d'air de traitement.

13. Appareil ménager (1) selon l'une quelconque des revendications 1 à 12, dans lequel le réservoir de liquide (21) comprend en outre un capteur de niveau de remplissage (26), dans lequel le capteur de niveau de remplissage (26) est placé à un niveau de remplissage prédéterminé (25) à l'intérieur du réservoir de liquide (21).

14. Appareil ménager (1) selon l'une quelconque des revendications 1 à 13, dans lequel le réservoir de liquide (21) comprend en outre un élément d'introduction d'air (20).

15. Procédé de fonctionnement d'un appareil ménager (1) avec un moyen pour générer un courant d'air (24) et un générateur d'aérosol (22) comprenant au moins un nébuliseur (28) et un réservoir de liquide (21) ayant au moins une surface intérieure active par catalyse contenant un polyoxométallate (17), dans lequel le polyoxométallate possède la Formule (I), (II) ou (III),
A₃PZ₄O₂₄ (I)
A_{q+3}PV_{q}Z₁₂-_{q}O₄₀ (II)
A₆P₂Z₁₈O₆₂ (III)
dans lequel Z est sélectionné parmi Mo ou W,
q est égal à 0, 1, 2 ou 3, et
A est choisi parmi un ou plusieurs cations sélectionnés parmi le groupe constitué par (XR¹R²R³R⁴)⁺, dans lequel au moins l'un des groupes R¹, R², R³ et R⁴ est un groupe alkyle, alkyloxoalkyle ou cycloalkyle substitué ou non substitué qui peut contenir au moins un hétéroatome sélectionné parmi O, S, N ou P, ayant au moins cinq atomes de carbone et dans lequel X est sélectionné parmi l'azote et le phosphore, **caractérisé en ce qu'**il comprend les étapes suivantes :
(a) remplir le réservoir de liquide (21) avec un liquide aqueux jusqu'à un niveau de remplissage prédéterminé (25) pour amener le liquide aqueux en contact avec l'au moins une surface intérieure active par catalyse contenant un polyoxométallate (17) ;
(b) nébuliser au moins une partie du liquide aqueux à l'intérieur du réservoir de liquide (21) avec l'au moins un nébuliseur (28) afin de créer un aérosol dans le réservoir de liquide (21) au-dessus du niveau de remplissage prédéterminé (25) ; et
(c) générer un courant d'air afin de diriger l'aérosol formé à l'étape (b) vers des surfaces dans l'appareil ménager (1).
